# EUROPEAN PATENT SPECIFICATION

(11) **EP 2 353 011 B1**
(45) Date of publication and mention of the grant of the patent: **04.03.2020**
(21) Application number: 09736845.0
(22) Date of filing: 07.10.2009
(51) Int. Cl.: G01N 33/68, G01N 33/574, G01N 33/74

(54) **BIOMARKER FOR THE PREDICTION OF FIRST ADVERSE EVENTS**
BIOMARKER ZUR PROGNOSE ERSTER UNERWÜNSCHTER EREIGNISSE
BIOMARQUEUR POUR LA PRÉDICTION D'EFFETS INDÉSIRABLES

(30) Priority: 07.10.2008 EP 08166038
(43) Date of publication of application: 10.08.2011
(73) Proprietor: B.R.A.H.M.S GmbH, 16761 Hennigsdorf (DE); The General Hospital Corporation, Boston, MA 02114 (US)
(72) Inventor: BERGMANN, Andreas, 12351 Berlin (DE); STRUCK, Joachim, 13465 Berlin (DE); MELANDER, Olle, 20502 Malmö (SE); NEWTON-CHEH, Christopher, 02114 Boston MA (US); WANG, Thomas J, 02114 Boston MA (US)
(74) Representative: Vossius & Partner Patentanwälte Rechtsanwälte mbB
(86) International application number: PCT/EP2009/007301
(87) International publication number: WO 2010/040564

(56) References cited:
- DE-A1-102006 034 142
- DE-A1-102006 052 916
- DE-A1-102006 060 112
- MORGENTHALER N G ET AL: "Measuremnet of midregional proadrenomedullin in plasma with an immunoluminometric assay" CLINICAL CHEMISTRY, AMERICAN ASSOCIATION FOR CLINICAL CHEMISTRY, WASHINGTON, DC, vol. 51, no. 10, 1 January 2005 (2005-01-01), pages 1823-1829, XP002480767 ISSN: 0009-9147 cited in the application
- CHRIST-CRAIN MIRJAM ET AL: "Mid-regional pro-adrenomedullin as a prognostic marker in sepsis: an observational study" CRITICAL CARE, BIOMED CENTRAL LTD., LONDON, GB, vol. 9, no. 6, 15 November 2005 (2005-11-15), pages R816-R824, XP021012417 ISSN: 1364-8535
- GEGENHUBER ET AL: "Comparative Evaluation of B-Type Natriuretic Peptide, Mid-Regional Pro-A-type Natriuretic Peptide, Mid-Regional Pro-Adrenomedullin, and Copeptin to Predict 1-Year Mortality in Patients With Acute Destabilized Heart Failure" JOURNAL OF CARDIAL FAILURE, CHURCHILL LIVINGSTONE, NAPERVILLE, IL, US, vol. 13, no. 1, 3 March 2007 (2007-03-03), pages 42-49, XP005910325 ISSN: 1071-9164
- KITAMURA KAZUO ET AL: "Adrenomedullin and PAMP: discovery, structures, and cardiovascular functions." MICROSCOPY RESEARCH AND TECHNIQUE 1 APR 2002, vol. 57, no. 1, 1 April 2002 (2002-04-01), pages 3-13, XP002516053 ISSN: 1059-910X
- MELANDER ET AL: JAMA, vol. 302, no. 1, 1 July 2009 (2009-07-01), pages 49-57, XP009126780
- AL-OMARI ET AL: AMERICAN JOURNAL OF HYPERTENSION, vol. 22, no. 8, 1 August 2009 (2009-08-01) , pages 862-866, XP009126781

### SEQUENCE LISTING

<110> BRAHMS AKTIENGESELLSCHAFT
<120> Biomarker for the Prediction of First Adverse Events
<130> B60403PCT
<160> 5
<170> PatentIn version 3.3
<210> 1
   <211> 185
   <212> PRT
   <213> Homo sapiens
<400> 1
   Met Lys Leu Val Ser Val Ala Leu Met Tyr Leu Gly Ser Leu Ala Phe
   1               5                   10                  15
   Leu Gly Ala Asp Thr Ala Arg Leu Asp Val Ala Ser Glu Phe Arg Lys
               20                  25                  30
   Lys Trp Asn Lys Trp Ala Leu Ser Arg Gly Lys Arg Glu Leu Arg Met
           35                  40                  45
   Ser Ser Ser Tyr Pro Thr Gly Leu Ala Asp Val Lys Ala Gly Pro Ala
       50                  55                  60
   Gln Thr Leu Ile Arg Pro Gln Asp Met Lys Gly Ala Ser Arg Ser Pro
   65                  70                  75                  80
   Glu Asp Ser Ser Pro Asp Ala Ala Arg Ile Arg Val Lys Arg Tyr Arg
                   85                  90                  95
   Gln Ser Met Asn Asn Phe Gln Gly Leu Arg Ser Phe Gly Cys Arg Phe
               100                 105                 110
   Gly Thr Cys Thr Val Gln Lys Leu Ala His Gln Ile Tyr Gln Phe Thr
           115                 120                 125
   Asp Lys Asp Lys Asp Asn Val Ala Pro Arg Ser Lys Ile Ser Pro Gln
       130                 135                 140
   Gly Tyr Gly Arg Arg Arg Arg Arg Ser Leu Pro Glu Ala Gly Pro Gly
   145                 150                 155                 160
   Arg Thr Leu Val Ser Ser Lys Pro Gln Ala His Gly Ala Pro Ala Pro
                   165                 170                 175
   Pro Ser Gly Ser Ala Pro His Phe Leu
               180                 185
<210> 2
   <211> 164
   <212> PRT
   <213> Homo sapiens
<400> 2
   Ala Arg Leu Asp Val Ala Ser Glu Phe Arg Lys Lys Trp Asn Lys Trp
   1 5 10 15
   Ala Leu Ser Arg Gly Lys Arg Glu Leu Arg Met Ser Ser Ser Tyr Pro
   20 25 30
   Thr Gly Leu Ala Asp Val Lys Ala Gly Pro Ala Gln Thr Leu Ile Arg
   35 40 45
   Pro Gln Asp Met Lys Gly Ala Ser Arg Ser Pro Glu Asp Ser Ser Pro
   50 55 60
   Asp Ala Ala Arg Ile Arg Val Lys Arg Tyr Arg Gln Ser Met Asn Asn
   65 70 75 80
   Phe Gln Gly Leu Arg Ser Phe Gly Cys Arg Phe Gly Thr Cys Thr Val
   85 90 95
   Gln Lys Leu Ala His Gln Ile Tyr Gln Phe Thr Asp Lys Asp Lys Asp
   100 105 110
   Asn Val Ala Pro Arg Ser Lys Ile Ser Pro Gln Gly Tyr Gly Arg Arg
   115 120 125
   Arg Arg Arg Ser Leu Pro Glu Ala Gly Pro Gly Arg Thr Leu Val Ser
   130 135 140
   Ser Lys Pro Gln Ala His Gly Ala Pro Ala Pro Pro Ser Gly Ser Ala
   145 150 155 160
   Pro His Phe Leu
<210> 3
   <211> 20
   <212> PRT
   <213> Homo sapiens
<400> 3
   Ala Arg Leu Asp Val Ala Ser Glu Phe Arg Lys Lys Trp Asn Lys Trp
   1 5 10 15
   Ala Leu Ser Arg
   20
<210> 4
   <211> 40
   <212> PRT
   <213> Homo sapiens
<400> 4
   Glu Leu Arg Met Ser Ser Ser Tyr Pro Thr Gly Leu Ala Asp Val Lys
   1 5 10 15
   Ala Gly Pro Ala Gln Thr Leu Ile Arg Pro Gln Asp Met Lys Gly Ala
   20 25 30
   Ser Arg Ser Pro Glu Asp Ser Ser
   35 40
<210> 5
   <211> 52
   <212> PRT
   <213> Homo sapiens
<400> 5
   Tyr Arg Gln Ser Met Asn Asn Phe Gln Gly Leu Arg Ser Phe Gly Cys
   1 5 10 15
   Arg Phe Gly Thr Cys Thr Val Gln Lys Leu Ala His Gln Ile Tyr Gln
   20 25 30
   Phe Thr Asp Lys Asp Lys Asp Asn Val Ala Pro Arg Ser Lys Ile Ser
   35 40 45
   Pro Gln Gly Tyr
   50

## Description

The present invention concerns the prediction of a first adverse event in healthy subjects, diagnostic assays and their uses as defined by the claims.

Effective cardiovascular prevention relies on the accurate identification of individuals at risk, but cardiovascular events frequently occur in individuals with a low burden of traditional risk factors (Khot UN, et al.: Prevalence of conventional risk factors in patients with coronary heart disease. JAMA. 2003;290:898-904). As a result, the potential use of circulating biomarkers to augment conventional risk assessment has attracted increasing attention in recent years. However, prior studies have reached differing conclusions regarding the utility of biomarkers for cardiovascular risk prediction. Some reports indicate that biomarkers such as C-reactive protein (CRP) and N-terminal pro-B-type natriuretic peptide (NT-proBNP) aid risk prediction (Ridker PM, et al.: Development and validation of improved algorithms for the assessment of global cardiovascular risk in women: the Reynolds Risk Score. JAMA. 2007;297:611-619; Zethelius B, et al.: Use of multiple biomarkers to improve the prediction of death from cardiovascular causes. N Engl J Med. 2008;358:2107-2116), whereas other studies conclude that such biomarkers contribute relatively little incremental information (Folsom AR, et al.: An assessment of incremental coronary risk prediction using C-reactive protein and other novel risk markers: the atherosclerosis risk in communities study. Arch Intern Med. 2006;166:1368-1373; Wang TJ, et al.: Multiple biomarkers for the prediction of first major cardiovascular events and death. N Engl J Med. 2006;355:2631-2639).

A number of factors influence how well biomarkers predict outcomes, including the population studied and the specific biomarkers selected. Studies focusing on elderly or high-risk populations often yield favourable estimates of biomarker performance (Zethelius B, et al.: Use of multiple biomarkers to improve the prediction of death from cardiovascular causes. N Engl J Med. 2008;358:2107-2116; Blankenberg S, et al.: Comparative impact of multiple biomarkers and N-Terminal pro-brain natriuretic peptide in the context of conventional risk factors for the prediction of recurrent cardiovascular events in the Heart Outcomes Prevention Evaluation (HOPE) Study; Circulation, 2006; 114:201-208), but the greatest need for new risk markers exists in low to intermediate-risk populations, for which the data are most conflicting (de Lemos JA, Lloyd-Jones DM: Multiple biomarker panels for cardiovascular risk assessment. N Engl J Med. 2008; 358:2172-2174). With regard to the statistical criteria for evaluating new biomarkers, it is widely accepted that basic association measures such as hazards ratios or odds ratios are insufficient to assess prognostic utility, but there is debate over what measures to use (Cook NR.: Use and misuse of the receiver operating characteristic curve in risk prediction. Circulation. 2007;115:928-935). Newer metrics assess how well biomarkers assign patients to clinical risk categories (Ridker PM, et al.: Development and validation of improved algorithms for the assessment of global cardiovascular risk in women: the Reynolds Risk Score. JAMA. 2007; 297:611-619; Pencina MJ, et al.: Evaluating the added predictive ability of a new marker: from area under the ROC curve to reclassification and beyond. Stat Med. 2008; 27:157-172), but only a few studies have incorporated such metrics (Zethelius B, et al.: Use of multiple biomarkers to improve the prediction of death from cardiovascular causes. N Engl J Med. 2008;358:2107-2116). Another important consideration is the selection of biomarkers. Although several biomarkers consistently predict cardiovascular events after adjustment for traditional risk factors (Vasan RS.: Biomarkers of cardiovascular disease: molecular basis and practical considerations. Circulation; 2006; 113:2335-2362), few primary prevention studies have incorporated multiple informative biomarkers simultaneously, an approach that has the greatest prospect of providing incremental information (de Lemos JA, Lloyd-Jones DM: Multiple biomarker panels for cardiovascular risk assessment. N Engl J Med. 2008; 358:2172-2174).

The peptide Adrenomedullin (ADM) was first described in 1993 (Kitamura et al. (1993), Biochem. Biophys. Res. Commun. 192:553-560) as a novel hypotensive peptide comprising 52 amino acids, which had been isolated from a human pheochromocytoma. In the same year, cDNA coding for a precursor peptide comprising 185 amino acids and the complete amino acid sequence of this precursor peptide were also described (Kitamura et al. (1993), Biochem. Biophys. Res. Commun. 194:720-725). The precursor peptide, which comprises, inter alia, a signal sequence of 21 amino acids at the N-terminus, is referred to as "pre-pro-Adrenomedullin" (pre-pro-ADM).

The ADM peptide comprises amino acids 95 to 146 of pre-pro-ADM, from which it is formed by proteolytic cleavage. Some peptide fragments of those formed in the cleavage of the pre-proADM have been characterized in detail, in particular the physiologically active peptides adrenomedullin (ADM) and "PAMP", a peptide comprising 20 amino acids (22-41) which follow the 21 amino acids of the signal peptide in pre-Pro-ADM. Another fragment of unknown function and high ex vivo stability is midregional proAdrenomedullin (MR-proADM) (Struck et al. (2004), Peptides 25(8):1369-72), for which a reliable quantification method has been developed (Morgenthaler et al. (2005), Clin. Chem. 51(10):1823-9).

The discovery and characterization of ADM in 1993 triggered intensive research activity and a flood of publications, the results of which have recently been summarized in various review articles, in the context of the present description, reference is being made in particular to the articles to be found in an issue of "Peptides" devoted to ADM (Peptides 22 (2001)), in particular (Takahashi (2001), Peptides 22, 1691 and Eto (2001), Peptides 22, 1693-1711). The subject is further reviewed in Hinson et al. (Hinson et al. (2000), Endocr. Rev. 21 (2), 138-167). ADM may be regarded as a polyfunctional regulatory peptide. It is released into the circulation in an inactive form extended by a C-terminal glycine (Kitamura et al. (1998), Biochem. Biophys. Res. Commun. 244 (2), 551-555).

ADM is an effective vasodilator. The hypotensive effect has been associated particularly with peptide segments in the C-terminal part of ADM. Peptide sequences of the N-terminus of ADM on the other hand exhibit hypertensive effects (Kitamura et al. (2001), Peptides 22, 1713-1718).

DE 102006052916 A1 discloses a method of diagnosis and risk stratification of diabetes mellitus based on midregional pro-adrenomedullin (MR-proADM). The diagnostic marker CT-proADM and its uses in risk stratification of diseases including the cardiovascular system is disclosed in DE 102006060112 A1. Further, DE 102006034142 A1 discloses methods for controlling the therapy of patients suffering from cardiac insufficiency by means of the determination of threshold values of vasoactive peptides in vitro. Morgenthaler et al., (2005), Clinical Chemistry, Vol. 51(10), 1823-1829, describes a sandwich immunoluminometric assay for MR-proADM. Increased MR-proADM concentrations were detected in patients with sepsis and with cardiovascular diseases. Christ-Crain et al., (2005), Critical Care, Vol. 9(6), R816-R824 reports on MR-proADM as a prognostic marker in sepsis. Gegenhuber et al., (2007), J. Cardial Failure, Vol. 13(1), 42-49, reports on the role of B-type natriuretic peptide, mid-regional pro-A-type natriuretic peptide, mid-regional pro-adrenomedullin and copeptin to predict 1-year mortality in patients with acute destabilized heart failure. Further, Kitamura et al., (2002), Microscopy Research and Technique, Vol. 57(1), 3-13, describes the physiological and pathological roles of adrenomedullin (AM) and proadrenomedullin N-terminal 20 peptide (PAMP) in cardiovascular diseases.

Subject of the present invention is a method for predicting the risk of getting a first adverse event in a healthy subject or identifying a healthy subject having an enhanced risk for getting a first adverse event comprising:
- determining the level of Pro-Adrenomedullin having amino acid sequence SEQ ID NO:2 or a fragment thereof selected from the group consisting of PAMP that is SEQ ID NO: 3, MR-proADM that is SEQ ID NO: 4 and ADM that is SEQ ID NO: 5 in a sample obtained from said subject; and
- using said level of Pro-Adrenomedullin or the fragment thereof for the prediction of the first adverse event or inferring from it a risk for getting the first adverse event,
wherein the use of said level of Pro-Adrenomedullin or the fragment thereof comprises comparing said level of Pro-Adrenomedullin or the fragment thereof to a predetermined threshold level being the 75^{th}, 90^{th}, 95^{th} or 99^{th} percentile of a normal population, whereby, when said level of Pro-Adrenomedullin or the fragment thereof exceeds said predetermined threshold level, the first adverse event is predicted in the healthy subject or the healthy subject having an enhanced risk for getting the first adverse event is identified and
wherein said first adverse event is a coronary or cardiovascular event, wherein the coronary event is defined as fatal or non-fatal acute coronary syndromes including myocardial infarction, or death due to ischemic heart disease, wherein cardiovascular event is defined as fatal or non-fatal acute coronary syndromes including myocardial infarction, fatal or non-fatal stroke, or death due to cardiovascular disease, and, wherein the healthy subject is a subject who has not previously had a cardiovascular or a coronary event and who is not having an acute infectious disease and who does not suffer from diabetes and/or hypertension and
wherein said sample is selected from the group consisting of a blood sample, a serum sample and a plasma sample.

In one aspect this adverse event may be the first which has ever been diagnosed for this subject. In one case a first event may be an e.g. coronary event which means that this subject has never had before a coronary event. In an aspect as defined in the claims the risk of getting a first adverse event is predicted and a subject is identified which has an advanced risk for getting a first adverse event.

In the context of the present disclosure, the term "Pro-Adrenomedullin" and the term "Pro-Adrenomedullin or fragments thereof' refer to either the entire molecule of Pro-Adrenomedullin or fragments thereof selected from the group consisting of PAMP that is depicted in SEQ ID NO: 3, MR-proADM that is depicted in SEQ ID NO: 4 and ADM that is depicted in SEQ ID NO: 5. Pro-Adrenomedullin refers to either the entire molecule of Pro-Adrenomedullin or the above described fragments thereof with the exception of mature Adrenomedullin. Pro-Adrenomedullin refers to either the entire molecule of Pro-Adrenomedullin or the above described fragments thereof with the exception of mature Adrenomedullin or fragments of mature Adrenomedullin. Thus, in one aspect "determining the level of Pro-Adrenomedullin or the above described fragments thereof' refers to determining the level of Pro-Adrenomedullin or fragments thereof, wherein the level of mature Adrenomedullin and/or fragments of mature Adrenomedullin is not determined.

The amino acid sequence of the precursor peptide of Adrenomedullin (pre-pro-Adrenomedullin) is given in Fig. 1 (SEQ ID NO:1). Pro-Adrenomedullin relates to amino acid residues 22 to 185 of the sequence of pre-pro-Adrenomedullin. The amino acid sequence of pro-Adrenomedullin (pro-ADM) is given in Fig. 2 (SEQ ID NO:2). The pro-ADM N-terminal 20 peptide (PAMP) relates to amino acid residues 22-41 of pre-pro-ADM. The amino acid sequence of PAMP is given in Fig. 3 (SEQ ID NO:3). MR-pro-Adrenomedullin (MR-pro-ADM) relates to amino acid residues 45-92 of pre-pro-ADM. The amino acid sequence of MR-pro-ADM is provided in Fig. 4 (SEQ ID NO:4). The amino acid sequence of mature Adrenomedullin (ADM) is given in Fig. 5 (SEQ ID NO:5).

As mentioned herein in the context of proteins or peptides, the term "fragment" refers to smaller proteins or peptides derivable from larger proteins or peptides, which hence comprise a partial sequence of the larger protein or peptide. Said fragments are derivable from the larger proteins or peptides by saponification of one or more of its peptide bonds and are selected from the group consisting of PAMP that is depicted in SEQ ID NO: 3, MR-proADM that is depicted in SEQ ID NO: 4 and ADM that is depicted in SEQ ID NO: 5.

Further, the term "level" in expressions such as "level of a protease", "analyte level" and similar expressions, refers to the quantity of the molecular entity mentioned in the respective context, or in the case of enzymes it can also refer to the enzyme activity.

An adverse event is defined as an event compromising the health of an individual. Said adverse event may be selected from the group comprising a coronary event, cardiovascular event, death, heart failure and hypertension. The term "diabetes" in the context of this disclosure is diabetes mellitus unless otherwise stated. Described is diabetes mellitus type 1 or type 2 diabetes mellitus. As defined in the claims, the first adverse event is a first coronary event or a first cardiovascular event, wherein the healthy subject is a subject who has not previously had a cardiovascular or a coronary event and who is not having an acute infectious disease and does not suffer from diabetes and/or hypertension.

Coronary events are defined as fatal or non-fatal acute coronary syndromes including myocardial infarction, or death due to ischemic heart disease.

Cardiovascular events are defined as fatal or non-fatal acute coronary syndromes including myocardial infarction, fatal or non-fatal stroke, or death due to cardiovascular disease.

As defined in the claims the method is for predicting a first adverse event in a healthy subject or identifying a healthy subject having an enhanced risk for getting a first adverse event, wherein said adverse event is a coronary or cardiovascular event such as a coronary event.

Said healthy subject is a subject with a low burden of traditional risk factors selected from the group comprising: cigarette smoking (defined as any smoking within the past year), diabetes, hyperlipidemia, hypertension, high body mass index (BMI) (i.e. preferably said subject has a BMI of below 30, preferably below 28, more preferably below 27, even more preferably below 26 and most preferably below 25), male gender, antihypertensive treatment, and age (the risk increases with age; preferably said subject has an age of below 55, preferably below 60, more preferably below 65, even more preferably below 70 and most preferably below 75).

"Healthy" or "apparently healthy", as used herein, relates to individuals who have not previously had or alternatively are not aware of having had a cardiovascular or a coronary event or heart failure, and who are not having an acute infectious disease. Healthy or apparently healthy relates to individuals who have not previously had a cardiovascular or a coronary event or heart failure, and who are not having an acute infectious disease.

Thus, as defined in the claims, the "healthy" or "apparently healthy" subject is a subject who has not previously had or alternatively is not aware of having had a cardiovascular or a coronary event. It is further disclosed that the "healthy" or "apparently healthy" subject does not suffer from diabetes, and/or hypertension.

The apparently healthy subject does not show any symptoms of any disease.

In one aspect, the adverse event is a coronary event and the "healthy" or "apparently healthy" subject may suffer from hyperlipidemia. In another aspect, the adverse event is a cardiovascular event and the "healthy" or "apparently healthy" subject may suffer from hyperlipidemia.

In yet another aspect, the adverse event is myocardial infarction and the "healthy" or "apparently healthy" subject is suffering from hyperlipidemia. In a further aspect, the adverse event is myocardial infarction and the "healthy" or "apparently healthy" subject does not show any symptoms of any disease.

In yet another aspect, the adverse event is death due to ischemic heart disease and the "healthy" or "apparently healthy" subject is suffering from hyperlipidemia. In a further aspect, the adverse event is ischemic heart disease and the "healthy" or "apparently healthy" subject does not show any symptoms of any disease.

In general, however, in case the "healthy" or "apparently healthy" subject is suffering from a particular disease, the first adverse event according to the present invention and as defined herein cannot be this particular disease.

The subgroups of healthy and apparently healthy subjects are according to this disclosure the same subgroup of subjects as an apparently healthy person is a person which has not yet been diagnosed as having or having had an event or condition comprising the health of said subject which means that an apparently healthy person is a person which is considered to be healthy.

In one aspect of the method of the invention as defined in the claims the level of Pro-Adrenomedullin or a fragment thereof selected from the group consisting of PAMP that is SEQ ID NO: 3, MR-proADM that is SEQ ID NO: 4 and ADM that is SEQ ID NO: 5 is determined and used as a single marker.

In another aspect of the invention the prediction of a first adverse event in a subject or the identification of a subject having an enhanced risk for getting a first adverse event is improved by additionally determining and using a laboratory parameter selected from the group consisting of: CRP, LpLA2, Cystatin C, ANP, proANP, NT-proANP, MR-proANP, BNP, proBNP, NT-proBNP, triglycerides, HDL cholesterol or subfractions thereof, LDL cholesterol or subfractions thereof, GDF15, ST2, Procalcitonin, Pro-Vasopressin including copeptin, vasopressin and neurophysin, Pro-Endothelin-1, CT-proET-1, NT-proET-1, big-Endothelin-1 and Endothelin-1.

As mentioned herein in the context of CRP, LpLA2, Cystatin C, ANP, BNP, proANP, NT-proANP, MR-proANP, proBNP, NT-proBNP, GDF15, ST2, Procalcitonin, Pro-Vasopressin including copeptin, vasopressin and neurophysin, Pro-Endothelin-1, CT-proET-1, NT-proET-1, big-Endothelin-1 and Endothelin-1, these terms refer to the entire molecule.

The term "additionally determining" does not imply, albeit not exclude, that such determinations are technically combined. The term "additionally using" is defined as any kind of mathematical combination of parameters - be it laboratory and/or clinical parameters - that yield a description of a subject's risk to experience an adverse event or identifying a subject having an enhanced risk for getting an adverse event. One example of such mathematical combination is the Cox proportional hazards analysis, from which a subject's risk to experience an adverse event can be derived, but other methods maybe used as well.

Described is that the level of marker for the individual is to be compared with a predetermined value. The predetermined value can take a variety of forms. It can be single cut-off value, such as for instance a median or mean or the 75^{th}, 90^{th}, 95^{th} or 99^{th} percentile of a population. It can be established based upon comparative groups, such as where the risk in one defined group is double the risk in another defined group. It can be a range, for example, where the tested population is divided equally (or unequally) into groups, such as a low-risk group, a medium-risk group and a high-risk group, or into quartiles, the lowest quartile being individuals with the lowest risk and the highest quartile being individuals with the highest risk.

The predetermined value can vary among particular populations selected, depending on their habits, ethnicity, genetics etc. For example, a "healthy" or "apparently healthy", non-smoker population (no detectable disease and no prior history of a cardiovascular disorder) might have a different 'normal' range of markers than a smoking population or a population the members of which have had a prior cardiovascular disorder. Accordingly, the predetermined values selected may take into account the category in which an individual falls. Appropriate ranges and categories can be selected with no more than routine experimentation by those of ordinary skill in the art.

The level of the above-mentioned marker can be obtained by any art recognized method. Typically, the level is determined by measuring the level or activity of the marker in a body fluid, for example, blood, lymph, or saliva. The level can be determined by immunoassays or other conventional techniques for determining the level of the marker. Recognized methods include sending samples of a patient's body fluid to a commercial laboratory for measurement, but also performing the measurement at the point-of-care.

A specific combination of markers seems to be especially valuable for predicting an adverse event in a subject or identifying a subject having an enhanced risk for getting an adverse event.

Thus, in one aspect of the invention as defined in the claims only the level of the following marker is determined and used: proBNP, CRP, or LpLA2 in combination with Pro-Adrenomedullin or fragments thereof. This specific combination seems to be especially valuable wherein a first cardiovascular event is predicted in a subject or a subject is identified having an enhanced risk for getting a first cardiovascular event.

Alternatively or additionally, determined levels of Pro-Adrenomedullin or fragments thereof may be combined with clinical parameters. Thus, in another preferred embodiment of the invention as defined in the claims the prediction of a first adverse event in a healthy subject or the identification of a healthy subject having an enhanced risk for getting a first adverse event is improved by determining and using clinical parameters, in addition to Pro-Adrenomedullin or fragments thereof, selected from the group: age, gender, antihypertensive treatment, body mass index and current smoking.

Further, the prediction of an adverse event in a healthy subject or the identification of a healthy subject having an enhanced risk for getting an adverse event is improved by determining and using clinical and laboratory parameters e.g. biomarker, in addition to Pro-Adrenomedullin or fragments thereof, selected from the aforementioned groups.

In another aspect of the method according to the invention as defined in the claims only the level of the following marker is determined and used: proBNP in combination with Pro-Adrenomedullin or fragments thereof. This specific combination seems to be especially valuable wherein a coronary event is predicted in a subject or a subject is identified having an enhanced risk for getting a coronary event especially a first coronary event.

The use of said level of Pro-Adrenomedullin or fragments thereof may comprise comparing said level of Pro-Adrenomedullin or fragments thereof to a threshold level, whereby, when said level of Pro-Adrenomedullin or fragments thereof exceeds said threshold level, a cardiovascular event or a first cardiovascular event is predicted in a subject or a subject having an enhanced risk for getting a (first) adverse event is identified.

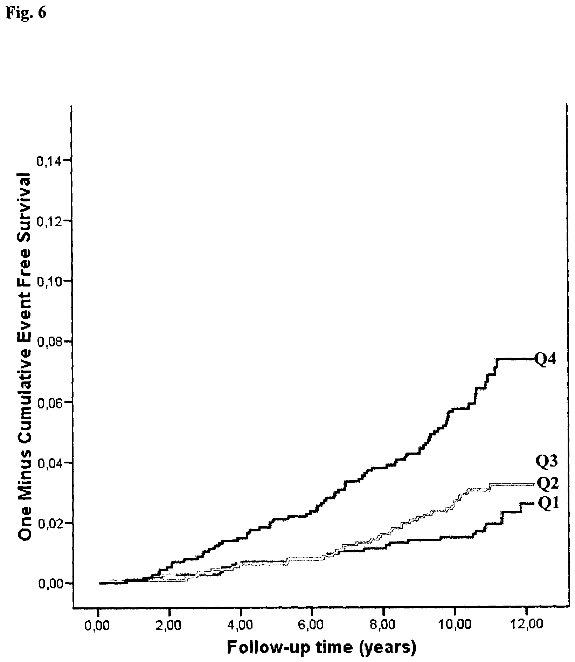

Such threshold level can be obtained for instance from a Kaplan-Meier analysis (Fig. 6), where the occurrence of coronary events over time in the investigated population is depicted according to MR-proADM quartiles of the population. According to this analysis, subjects with MR-proADM levels above the 75^{th} percentile, that is above 0.52 nmol/L in the investigated population, have a significantly increased risk for getting a first adverse coronary event. This result is further supported by Cox regression analysis with full adjustment for classical risk factors: The highest MR-proADM quartile (all subjects with MR-proADM levels above 0.52 nmol/L) versus all other subjects is highly significantly associated with increased risk (HR=1.6 95% CI 1.2-2.2) for getting a first adverse coronary event.

Other cut-off values are for instance the 90^{th}, 95^{th} or 99^{th} percentile of a normal population. By using a higher percentile than the 75^{th} percentile, one reduces the number of false positive subjects identified, but one might miss to identify subjects, who are at moderate, albeit still increased risk. Thus, one might adopt the cut-off value depending on whether it is considered more appropriate to identify most of the subjects at risk at the expense of also identifying "false positives", or whether it is considered more appropriate to identify mainly the subjects at high risk at the expense of missing several subjects at moderate risk.

Other mathematical possibilities to calculate an individual's risk by using the individual's MR-proADM value and other prognostic laboratory and clinical parameters are for instance the NRI (Net Reclassification Index) or the IDI (Integrated Discrimination Index). The indices can be calculated according to Pencina (Pencina MJ, et al.: Evaluating the added predictive ability of a new marker: from area under the ROC curve to reclassification and beyond. Stat Med. 2008;27:157-172).

Put simply, NRI (Net Reclassification Index) (based on CVD risk classes according to ATPIII risk algorithm, <10, 10-<20 and 20+ % 10-year risk) calculates if - by adding for example MR-proADM to the model of classical risk factors - one moves a significant number of subjects who in fact had an event to a higher risk class and subjects who did not have an event to lower risk class. IDI (Integrated Discrimination Index), does a similar thing but ignores the borders of<10, 10-<20 and 20+ % 10-year risk and instead calculates the "total integrated movement", i.e. does addition of MR-proADM move subjects who had an event upwards on a continuous risk scale and subjects who did not have event downwards on the continuous risk scale.

NRI has clinical relevance as crossing the "magical border" of 20% means patient should be treated as a secondary preventive patient and thus means something to treatment (according to cardiology guidelines for prevention).

Further described is a method for predicting the risk of getting an adverse event in a healthy subject or identifying a subject having an enhanced risk for getting an adverse event according to any of the preceding claims, wherein the level of Pro-Adrenomedullin or fragments thereof selected from the group of sequences depicted in SEQ ID NOS: 3, 4 and 5, either alone or in conjunction with other prognostically useful laboratory or clinical parameters, is used for the prediction of a healthy subject's risk for getting an adverse event by a method which may be selected from the following alternatives:
Comparison with the median of the level of Pro-Adrenomedullin or the above-mentioned fragments thereof in an ensemble of pre-determined samples in a population of "healthy" or "apparently healthy" subjects,
Comparison with a quantile (e.g. the 75^{th}, 90^{th}, 95^{th} or 99^{th} percentile) of the level of Pro-Adrenomedullin or fragments thereof in an ensemble of pre-determined samples in a population of apparently healthy subjects,
Calculation based on Cox Proportional Hazards analysis or by using Risk index calculations such as the NRI (Net Reclassification Index) or the IDI (Integrated Discrimination Index).

Said sample is selected from the group consisting of a blood sample, a serum sample and a plasma sample, or an extract of any of the aforementioned samples.

In another aspect as defined in the claims the level of MR-proADM is measured. MR-proADM comprises amino acids 45-92 of Pre-Pro-ADM.

In another aspect of the invention as defined in the claims, the level of Pro-Adrenomedullin is measured using a diagnostic assay using one or more capture probes directed against one ore more epitopes located in amino acid positions 45-92 of Pre-pro-ADM.

As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably greater than 10⁸ M⁻¹.

In the context of the disclosure, "capture molecules" are molecules which may be used to bind target molecules or molecules of interest, i.e. analytes, from a sample. Capture molecules must thus be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. Capture molecules may for instance be selected from the group comprising a nucleic acid molecule, a carbohydrate molecule, a PNA molecule, a protein, an antibody, a peptide or a glycoprotein. Capture molecules may be antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof.

Detection methods may comprise immunoassays in various formats such as for instance radioimmunoassays, chemiluminescence- and fluorescence- immunoassays, Enzyme-linked immunoassays (ELISA), Luminex-based bead arrays, protein microarray assays, and rapid test formats such as for instance immunochromatographic strip tests. The assays can be homogenous or heterogeneous assays, competitive and non-competitive sandwich assays. The assay may be in the form of a sandwich assay, which is a noncompetitive immunoassay, wherein the molecule to be detected and/or quantified is bound to a first antibody and to a second antibody. The first antibody may be bound to a solid phase, e.g. a bead, a surface of a well or other container, a chip or a strip, and the second antibody is an antibody which is labeled, e.g. with a dye, with a radioisotope, or a reactive or catalytically active moiety. The amount of labeled antibody bound to the analyte is then measured by an appropriate method. The general composition and procedures involved with "sandwich assays" are well-established and known to the skilled person. (The Immunoassay Handbook, Ed. David Wild, Elsevier LTD, Oxford; 3rd ed. (May 2005), ISBN-13: 978-0080445267; Hultschig C et al., Curr Opin Chem Biol. 2006 Feb;10(1):4-10. PMID: 16376134).

The assay may comprise two capture molecules, preferably antibodies which are both present as dispersions in a liquid reaction mixture, wherein a first marking component is attached to the first capture molecule, wherein said first marking component is part of a marking system based on fluorescence- or chemiluminescence-quenching or amplification, and a second marking component of said marking system is attached to the second capture molecule, so that upon binding of both capture molecules to the analyte a measurable signal is generated that allows for the detection of the formed sandwich complexes in the solution comprising the sample.

Further, said marking system may comprise rare earth cryptates or rare earth chelates in combination with a fluorescence dye or chemiluminescence dye, in particular a dye of the cyanine type.

Fluorescence based assays may comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, auch as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine,Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

Chemiluminescence based assays may comprise the use of dyes, based on the physical principles described for chemiluminescent materials in Kirk-Othmer, Encyclopedia of chemical technology, 4th ed., executive editor, J. I. Kroschwitz; editor, M. Howe-Grant, John Wiley & Sons, 1993, vol.15, p. 518-562, including citations on pages 551-562. Preferred chemiluminescent dyes are Acridiniumesters.

In an aspect, a MR-proADM assay is used having a detection limit below 0.3 nmol/L and an inter-assay precision of <30% CV in the normal range for predicting a first adverse event in a healthy subject or identifying a healthy subject having an enhanced risk for getting a first adverse event.

Further disclosed is the use of a capture probe, e.g. antibody directed against Pro-Adrenomedullin or fragments thereof for predicting a first adverse event in a healthy subject or identifying a healthy subject having an enhanced risk for getting a first adverse event.

Disclosed is the use of one or more antibodies which are directed against an epitope included in the amino acids positions 45-92 of Pre-ProADM.

### Examples

The following examples outline the present invention in greater detail but shall not be understood as limiting the subject matter of the present invention. Examples which do not fall under the subject-matter of the appended claims do not form part of the present invention.

### METHODS

### Study population

The Malmö Diet and Cancer (MDC) study is a community-based, prospective epidemiologic cohort of 28,449 persons enrolled between 1991 and 1996. From this cohort, 6,103 persons were randomly selected to participate in the MDC Cardiovascular Cohort, which was designed to investigate the epidemiology of atherosclerosis. Complete data on cardiovascular risk factors and plasma biomarkers were available on 4,707 subjects. After excluding participants with prevalent cardiovascular disease at baseline, there were 4,601 participants in the analysis of coronary events, and 4,483 participants in the analysis of all cardiovascular events.

### Clinical examination and assays

All participants underwent a medical history, physical examination, and laboratory assessment of standard cardiovascular risk factors. Blood pressure was measured using a mercury sphyngomanometer after 10 minutes of rest in the supine position. Diabetes mellitus was defined as a fasting blood glucose greater than 6.0 mmol/L, a self-reported physician diagnosis of diabetes, or use of anti-diabetic medication. Cigarette smoking was elicited by a self-administered questionnaire, with current cigarette smoking defined as any smoking within the past year. We measured fasting total cholesterol, HDL cholesterol, and triglycerides according to standard procedures at the Department of Clinical Chemistry, University Hospital Malmö. LDL cholesterol was calculated according to Friedewald's formula.

All participants had assessment of cardiovascular biomarkers using fasting plasma specimens that had been frozen at -80 °C immediately after collection. C-reactive protein was measured by high-sensitivity assay (Tina-quant CRP, Roche Diagnostics, Basel, Switzerland). LpPLA2 activity was measured in duplicate using [3H]-platelet activating factor as substrate (Persson M, et al.: Elevated Lp-PLA2 levels add prognostic information to the metabolic syndrome on incidence of cardiovascular events among middle-aged nondiabetic subjects. Arterioscler Thromb Vasc Biol. 2007;27:1411-1416). MR-proANP and MR-proADM were measured using immunoluminometric sandwich assays targeted against amino acids in the mid-regions of the respective peptide (BRAHMS, AG, Germany) (Morgenthaler NG, et al.: Immunoluminometric assay for the midregion of pro-atrial natriuretic peptide in human plasma. Clin Chem. 2004;50:234-236; Morgenthaler NG, et al.: Measurement of midregional proadrenomedullin in plasma with an immunoluminometric assay. Clin Chem. 2005;51:1823-1829). NT-proBNP was determined using the Dimension RxL N-BNP (Dade-Behring, Germany) (Di SF, et al.: Analytical evaluation of the Dade Behring Dimension RxL automated N-Terminal proBNP (NT-proBNP) method and comparison with the Roche Elecsys 2010. Clin Chem Lab Med. 2005;43:1263-1273). Cystatin C was measured using a particle-enhanced immuno-nephelometric assay (N Latex Cystatin C, Dade Behring, IL) (Shlipak MG, et al.: Cystatin C and the risk of death and cardiovascular events among elderly persons. N Engl J Med. 2005;352:2049-2060).

### Clinical endpoints

We examined two primary outcomes: coronary events and all cardiovascular events. The procedure for ascertaining outcome events has been detailed previously (Rosvall M, et al.: Incident coronary events and case fatality in relation to common carotid intima-media thickness. J Intern Med. 2005;257:430-437; Rosvall M, et al.: Incidence of stroke is related to carotid IMT even in the absence of plaque. Atherosclerosis. 2005;179:325-331). Coronary events were defined as fatal or non-fatal acute coronary syndromes including myocardial infarction, or death due to ischemic heart disease. All cardiovascular events were defined as fatal or non-fatal acute coronary syndromes including myocardial infarction, fatal or non-fatal stroke, or death due to cardiovascular disease. Events were identified through linkage of the 10-digit personal identification number of each Swedish citizen with two registries - the Swedish Hospital Discharge Register and the Swedish Cause of Death Register. Myocardial infarction was defined on the basis of *International Classification of Diseases* 9^{th} and 10^{th} Revisions (ICD9 and ICD10) codes 410 and 121, respectively. Death due to ischemic heart disease was defined on the basis of codes 412 and 414 (ICD9) or 122-123 and 125 (ICD10) in the Swedish Cause of Death Register. Fatal or nonfatal stroke was using codes 430, 431 and 434 (ICD9). Classification of outcomes using these registries has been previously validated (Engstrom G, et al.: Geographic distribution of stroke incidence within an urban population: relations to socioeconomic circumstances and prevalence of cardiovascular risk factors. Stroke. 2001;32:1098-1103). Follow-up for outcomes extended to December 31, 2003.

### Statistical analyses

Continuous biomarker variables with skewed distributions were log-transformed before analysis. We performed multivariable Cox proportional hazards models to examine the association between biomarkers and incident events. We confirmed that the proportionality of hazards assumption was met. Hazards ratios were expressed per 1 SD increment in the respective biomarker. Each biomarker was individually tested against each endpoint with adjustment for traditional risk factors (age, sex, systolic blood pressure, diastolic blood pressure, use of antihypertensive therapy, current smoking, diabetes, LDL cholesterol, HDL cholesterol, and body mass index). Next, all of the biomarkers showing a significant relationship with the outcome were included in a backward elimination model, with the traditional risk factors forced in. P<0.05 was used as the criterion for retaining biomarkers in this model.

To assess model discrimination, we calculated the c-statistic for models with traditional risk factors with and without biomarkers. The c-statistic is a generalization of the area under the receiver-operating-characteristic curve (Pencina MJ, et al.: Evaluating the added predictive ability of a new marker: from area under the ROC curve to reclassification and beyond. Stat Med. 2008;27:157-172). We used the incidence of coronary or cardiovascular events at 10 years as the outcome. To assess global calibration of the risk models, we calculated Hosmer-Lemeshow statistics for models with and without biomarkers. Non-significant p-values for the Hosmer-Lemeshow statistic denote good agreement between predicted and observed event rates across deciles of predicted risk.

We also evaluated the ability of biomarkers to reclassify risk across National Cholesterol Education Program Adult Treatment Panel III (ATP3) risk categories, following methods suggested previously (Ridker PM, et al.: Development and validation of improved algorithms for the assessment of global cardiovascular risk in women: the Reynolds Risk Score. JAMA. 2007;297:611-619; Pencina MJ, et al.: Evaluating the added predictive ability of a new marker: from area under the ROC curve to reclassification and beyond. Stat Med. 2008;27:157-172). Based on a traditional risk factor score, participants were initially classified as low, intermediate, or high risk if their predicted 10-year risk of a coronary event was <10%, 10% to <20%, or ≥20%, respectively. Participants were then allowed to be reclassified into different categories with the addition of the biomarker data. We assessed the number of participants reclassified, and also calculated the Net Reclassification Index (NRI) and Integrated Discrimination Index (IDI), as described by (Pencina MJ, et al.: Evaluating the added predictive ability of a new marker: from area under the ROC curve to reclassification and beyond. Stat Med. 2008;27:157-172). In the calculation of the NRI, individuals were considered accurately reclassified if they were reclassified to a higher category and subsequently developed a coronary event, or if they were reclassified to a lower category and remained event-free during follow-up. The IDI is an analogous measure of model performance, which improves when the risk model assigns higher probabilities to those who develop events and lower probabilities to those who remain event-free. In contrast to the NRI, the IDI is based on continuous probabilities, and thus improvement in the index does not rely on movement across risk category thresholds.

All analyses were performed using Stata software version 8.0 (StataCorp) except for the tests for the proportionality of hazards assumption which were performed using the survival package for R, and the c-statistics which were generated using the ROCR package for R (www.r-project.org). Tests were considered significant if the two-sided P-value was less than 0.05.

### RESULTS

Characteristics of the study sample are shown in **Table 1.** The mean age of the sample was 58 years.

### Prediction of cardiovascular events using single biomarkers

During a mean follow up of 10.6 years, first cardiovascular and first coronary events occurred in 288 and 169 participants, respectively. Results of Cox proportional hazards models for the association of individual biomarkers with cardiovascular events are shown in **Table 2.** After adjustment for traditional risk factors, 5 of 6 biomarkers (NT-proBNP, CRP, cystatin C, MR-proADM, LpPLA2) were significant predictors of incident cardiovascular events.

Several metrics were used to summarize the prognostic utility of adding individual biomarkers to classical risk factors (**Table 2**). A model based on classical risk factors had a c-statistic of 0.772, and the addition of individual biomarkers resulted in small increases in the c-statistic (<0.005). Models were well-calibrated, with Hosmer-Lemeshow p-values >0.05 whether or not biomarkers were included. Neither the NRI nor the IDI were significant for all biomarkers.

### Prediction of coronary events using single biomarkers

Results of Cox proportional hazards models for coronary events are shown in **Table 3.** When entered individually into a model with classical risk factors, 4 of the 6 biomarkers (NT-proBNP, MR-proANP, cystatin C, MR-proADM) were significant predictors of incident coronary events. Elevations in NT-proBNP and MR-proADM were associated with the highest hazards for coronary events, with adjusted hazards ratios per SD increment of 1.24 (95% confidence interval, 1.07-1.44) and 1.25 (95% confidence interval, 1.10-1.42), respectively.

The c-statistic associated with classical risk factors for predicting coronary events was 0.777. As with cardiovascular events, addition of individual biomarkers did not raise the c-statistic substantially (**Table 3**). Model calibration was good (Hosmer-Lemeshow p>0.05) with or without biomarkers, and the NRI was non-significant. The IDI was significant for MR-proADM (p=0.02), and borderline significant for NT-proBNP (p=0.05).

### Multiple biomarkers for cardiovascular and coronary events

Combinations of biomarkers were tested by entering the biomarkers as a set into prediction models for cardiovascular events and coronary events. Stepwise backward elimination was then used to remove less informative biomarkers, using p<0.05 as the criteria for retention. Three biomarkers were retained for prediction of cardiovascular events (NT-proBNP, CRP, and LpPLA2), and 2 biomarkers were retained for prediction of coronary events (NT-proBNP and MR-proADM). Results of multivariable Cox proportional hazards models are shown in Table 4, for both outcomes. Incorporation of the set of significant biomarkers into prediction models for cardiovascular and coronary events led to small increments (approximately 0.01) in the c-statistics. The NRI was not significant for either outcome. On the other hand, the IDI had p-values of 0.06 for cardiovascular events and 0.02 for coronary events.
**Tables 5** shows the number of participants reclassified using biomarkers for cardiovascular events (Panel A) and coronary events (Panel B), respectively. For cardiovascular events, use of biomarkers moved 273 participants (6%) into a higher or lower risk category. Only 39 partcipants (0.8%) were moved into the high risk category (10-year predicted risk ≥20%). For coronary events, 137 (3%) participants were reclassified into a higher or lower risk category, with only 22 (0.5%) moved into the high risk category.

Simple biomarker risk scores were constructed for each endpoint. Beta coefficients for the 3 biomarkers predicting cardiovascular events were similar in magnitude, as were the beta coefficients for the 2 biomarkers predicting coronary events. Thus, we elected to weight each of the biomarkers equally in the biomarker risk scores. For cardiovascular disease, each participant was assigned a score of 0, 1, 2, or 3, corresponding to the number of biomarkers that were elevated, defined as having a level more than 1 SD above the mean. In a similar fashion, each participant was assigned a score of 0, 1, or 2 for coronary events. **Figure 6** depicts the cumulative incidence of cardiovascular (**Panel A**) or coronary (**Panel B**) events, according to values of the biomarker risk scores.

**Table 1: Characteristics of the study sample (n=4,601)**

| Clinical variable | |
|---|---|
| Age, years | 58 ± 6 |
| Male gender, % | 40 |
| Systolic blood pressure, mm Hg | 141 ± 19 |
| Diastolic blood pressure, mm Hg | 87 ± 9 |
| Antihypertensive treatment, % | 16 |
| Body mass index, kg/m² | 25.7 ± 3.9 |
| LDL cholesterol, mmol/L | 4.2 ± 1.0 |
| HDL cholesterol, mmol/L | 1.4 ± 0.4 |
| Diabetes mellitus, % | 8 |
| Current smoking, % | 27 |
| MR-proADM, nmol/L | 0.46 ± 0.13 |
| MR-proANP, pmol/L | 66 (51-86) |
| NT-proBNP, pg/mL | 61 (34-110) |
| Cystatin C, mg/L | 0.78 ± 0.15 |
| CRP, mg/L | 1.3 (0.7-2.7) |
| LpPLA2 activity, nmol/min/mL | 44 (36-52) |

Normally distributed data are given as mean ± SD, skewed variables are given as median (IQR).

**Table 2: Individual biomarkers and incident cardiovascular events (n=4,481)**

| Biomarker | Adjusted HR | 95% CI | P | ΔC* | P_{NRI}† | P_{IDI}† |
|---|---|---|---|---|---|---|
| NT-proBNP | 1.18 | (1.05-1.33) | 0.007 | 0.004 | 0.21 | 0.06 |
| MR-proADM | 1.14 | (1.02-1.27) | 0.01 | 0.004 | 0.91 | 0.19 |
| MR-proANP | 1.12 | (0.99-1.26) | 0.06 | 0.001 | 0.78 | 0.26 |
| Cystatin C | 1.12 | (1.02-1.24) | 0.01 | 0.004 | 0.52 | 0.44 |
| LpPLA2 | 1.14 | (1.005-1.30) | 0.04 | 0.003 | 0.68 | 0.51 |
| CRP | 1.19 | (1.05-1.34) | 0.005 | 0.004 | 0.92 | 0.43 |

All hazards ratios are expressed per SD increment in biomarker concentration. Multivariable Cox proportional hazards models were adjusted for age, sex, antihypertensive treatment, systolic blood pressure, diastolic blood pressure, body mass index, diabetes, LDL, HDL, and current smoking. *Increment in C-statistic in a model with classical risk factors and the biomarker, compared with a classical risk factors alone (C=0.772). †P-value for increase in Net Reclassification Index (NRI) or integrated discrimination index (IDI) in a model with classical risk factors and the biomarker, compared with classical risk factors alone. CI, confidence interval; HR, hazards ratio.

**Table 3: Individual biomarkers and incident coronary events (n=4,601)**

| Biomarker | Adjusted HR | 95% CI | P | ΔC* | P_{NRI}† | P_{IDI}† |
|---|---|---|---|---|---|---|
| NT-proBNP | 1.24 | (1.07-1.44) | 0.005 | 0.004 | 0.70 | 0.05 |
| MR-proADM | 1.25 | (1.10-1.42) | <0.001 | 0.005 | 0.43 | 0.02 |
| MR-proANP | 1.21 | (1.04-1.41) | 0.01 | 0.0001 | 0.42 | 0.12 |
| Cystatin C | 1.15 | (1.05-1.28) | 0.005 | 0.004 | 0.55 | 0.30 |
| LpPLA2 | 1.07 | (0.91-1.26) | 0.31 | NA | NA | NA |
| CRP | 1.12 | (0.96-1.31) | 0.17 | NA | NA | NA |

All hazards ratios are expressed per SD increment in biomarker concentration. Multivariable Cox proportional hazards models were adjusted for age, sex, antihypertensive treatment, systolic blood pressure, diastolic blood pressure, body mass index, diabetes, LDL, HDL, and current smoking. *Increment in C-statistic in a model with classical risk factors and the biomarker, compared with a classical risk factors alone (C=0.777). †P-value for increase in Net Classification Index (NRI) or integrated discrimination index (IDI) in a model with classical risk factors and the biomarker, compared with classical risk factors alone. CI, confidence interval; HR, hazards ratio; NA, not analysed.

**Table 4: Multiple biomarkers and incident cardiovascular and coronary events**

| Biomarkers retained by backwards elimination | Adjusted HR | 95% CI | P | ΔC* | P_{NRI}† | P_{IDI}† |
|---|---|---|---|---|---|---|
| **First cardiovascular events** | | | | | | |
| NT-proBNP | 1.16 | (1.03-1.30) | 0.017 | 0.008 | 0.13 | 0.06 |
| CRP | 1.18 | (1.03-1.33) | 0.008 | | | |
| LpPLA2 | 1.15 | (1.01-1.30) | 0.03 | | | |
| **First coronary events** | | | | | | |
| NT-proBNP | 1.19 | (1.02-1.38) | 0.03 | 0.009 | 0.28 | 0.02 |
| MR-proADM | 1.21 | (1.06-1.38) | 0.004 | | | |

All hazards ratios are expressed per SD increment in biomarker concentration. Multivariable Cox proportional hazards models were adjusted for age, sex, antihypertensive treatment, systolic blood pressure, diastolic blood pressure, body mass index, diabetes, LDL, HDL, and current smoking. For each endpoint, all biomarkers shown were entered together into the Cox model. *Increment in C-statistic in a model with classical risk factors and the biomarker set. †P-value for increase in Net Classification Index (NRI) or integrated discrimination index (IDI) in a model with classical risk factors and the biomarker set, compared with classical risk factors alone. CI, confidence interval; HR, hazards ratio.

**Table 5: Reclassification of participants with addition of biomarkers to traditional cardiovascular risk factors**

| **Panel A: Cardiovascular Events** | | | | |
|---|---|---|---|---|
| **Model with traditional risk factors alone** | **Model with traditional risk factors and biomarkers** | | | |
| | **<10%** | **10% to <20%** | **≥20%** | **Total** |
| **<10%** | 3,728 | 103 | 1 | 3,832 |
| **10% to <20%** | 99 | 344 | 38 | 481 |
| **≥20%** | 0 | 32 | 138 | 170 |
| **Total** | 3,827 | 479 | 177 | 4,483 |

| **Panel B: Coronary Events** | | | | |
|---|---|---|---|---|
| **Model with traditional risk factors alone** | **Model with traditional risk factors and biomarkers** | | | |
| | **<10%** | **10% to <20%** | **≥20%** | **Total** |
| **<10%** | 4,265 | 52 | 0 | 4,317 |
| **10% to <20%** | 52 | 165 | 22 | 239 |
| **≥20%** | 0 | 11 | 34 | 45 |
| **Total** | 4,317 | 228 | 56 | 4,601 |

### Description of drawings

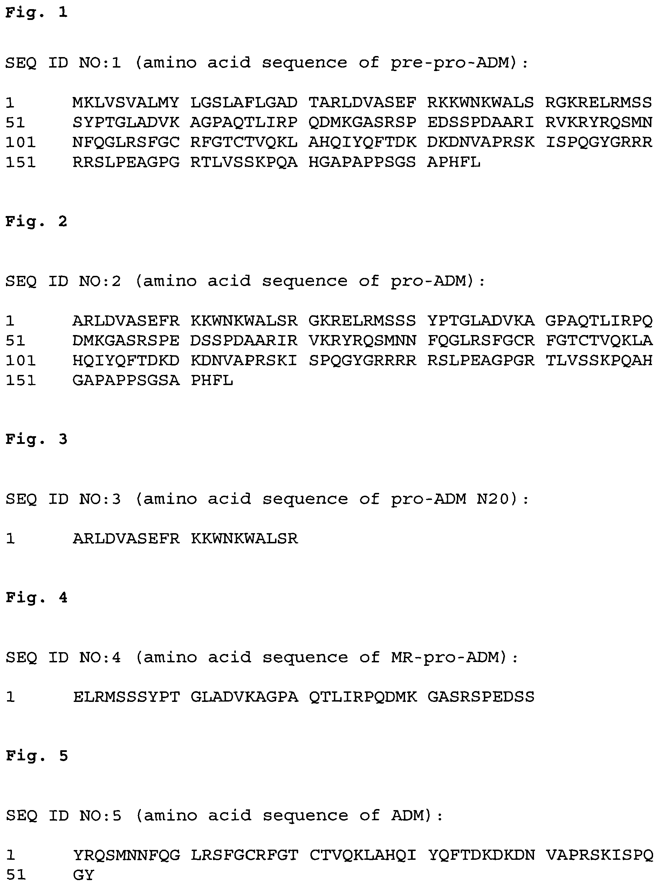

Fig 1: Amino acid sequence of the adrenomedullin (ADM) precursor peptide (pre-pro-ADM). Amino acids 1-21 form a signal peptide. Amino acids 22-41 form the pro-ADM N-20 terminal peptide (pro-ADM N20)). Amino acids 45-92 form the MR-proADM peptide. Mature ADM comprises amino acids 95-146. Amino acids 148-185 form the pro-ADM C-terminal fragment.
Fig 2: Amino acid sequence of the pro-adrenomedullin peptide (pro-ADM).
Fig 3: Amino acid sequence of the pro-adrenomedullin N-terminal 20 peptide (pro-ADM N20; PAMP). The PAMP peptide may have an amidated C-term.
Fig 4: Amino acid sequence of the MR pro-adrenomedullin (MR-pro-ADM).
Fig 5: Amino acid sequence of the mature adrenomedullin peptide (ADM). ADM peptide may have an amidated C-term and/or may be glycosylated.
Fig. 6: Kaplan-Meier-Plot for coronary events of the investigated population grouped in MR-proADM quartiles.

## Claims

1. A method for predicting the risk of getting a first adverse event in a healthy subject or identifying a healthy subject having an enhanced risk for getting a first adverse event comprising:
• determining the level of Pro-Adrenomedullin having amino acid sequence SEQ ID NO:2 or a fragment thereof selected from the group consisting of PAMP that is SEQ ID NO: 3, MR-proADM that is SEQ ID NO: 4 and ADM that is SEQ ID NO: 5 in a sample obtained from said subject; and
• using said level of Pro-Adrenomedullin or the fragment thereof for the prediction of the first adverse event or inferring from it a risk for getting the first adverse event,
wherein the use of said level of Pro-Adrenomedullin or the fragment thereof comprises comparing said level of Pro-Adrenomedullin or the fragment thereof to a predetermined threshold level being the 75^{th}, 90^{th}, 95^{th} or 99^{th} percentile of a normal population, whereby, when said level of Pro-Adrenomedullin or the fragment thereof exceeds said predetermined threshold level, the first adverse event is predicted in the healthy subject or the healthy subject having an enhanced risk for getting the first adverse event is identified and
wherein said first adverse event is a coronary or cardiovascular event, wherein the coronary event is defined as fatal or non-fatal acute coronary syndromes including myocardial infarction, or death due to ischemic heart disease, wherein cardiovascular event is defined as fatal or non-fatal acute coronary syndromes including myocardial infarction, fatal or non-fatal stroke, or death due to cardiovascular disease, and, wherein the healthy subject is a subject who has not previously had a cardiovascular or a coronary event and who is not having an acute infectious disease and who does not suffer from diabetes and/or hypertension and
wherein said sample is selected from the group consisting of a blood sample, a serum sample and a plasma sample.

2. A method according to claim 1, wherein the level of Pro-Adrenomedullin or a fragment thereof selected from the group consisting of PAMP that is SEQ ID NO: 3, MR-proADM that is SEQ ID NO: 4 and ADM that is SEQ ID NO: 5 is determined and used as a single marker.

3. A method according to claim 1, wherein the prediction of the first adverse event in the healthy subject or the identification of the healthy subject having an enhanced risk for getting the first adverse event is improved by additionally determining and using the level of at least one further marker selected from the group consisting of: CRP, LpLA2, Cystatin C, ANP, proANP, NT-proANP, MR-proANP, BNP, proBNP, NT-proBNP triglycerides, HDL cholesterol or subfractions thereof, LDL cholesterol or subfractions thereof, GDF15, ST2, Procalcitonin, Pro-Vasopressin including copeptin, vasopressin and neurophysin, Pro-Endothelin-1, CT-proET-1, NT-proET-1, big-Endothelin-1 and Endothelin-1.

4. A method according to claim 3, wherein only the level of the following marker is determined and used: proBNP, CRP or LpLA2 in combination with Pro-Adrenomedullin or a fragment thereof selected from the group consisting of PAMP that is SEQ ID NO: 3, MR-proADM that is SEQ ID NO: 4 and ADM that is SEQ ID NO: 5.

5. A method according to claim 4, wherein the first cardiovascular event is predicted in the healthy subject or the healthy subject is identified having an enhanced risk for getting the first cardiovascular event.

6. A method according to claim 3, wherein only the level of the following marker is determined and used: proBNP in combination with Pro-Adrenomedullin or a fragment thereof selected from the group consisting of PAMP that is SEQ ID NO: 3, MR-proADM that is SEQ ID NO: 4 and ADM that is SEQ ID NO: 5.

7. A method according to claim 6, wherein the first coronary event is predicted in the healthy subject or the healthy subject is identified having an enhanced risk for getting the first coronary event.

8. A method according to claims 1 to 7, wherein additionally at least one clinical parameter is determined selected from the group comprising: age, gender, antihypertensive treatment, body mass index, and current smoking.

9. A method according to any of the preceding claims, wherein the level of MR-proADM is measured using a diagnostic assay comprising one or more capture probes directed against one or more epitopes located in amino acid positions 45-92 of Pre-pro-ADM.

10. Use of an MR-proADM assay in the methods of any of claims 1 to 9.

11. Use of a capture probe directed against Pro-Adrenomedullin or a fragment thereof selected from the group consisting of PAMP that is SEQ ID NO: 3, MR-proADM that is SEQ ID NO: 4 and ADM that is SEQ ID NO: 5 for predicting the first adverse event in the healthy subject or identifying the healthy subject having an enhanced risk for getting the first adverse event according to the methods of any of claims 1 to 9, or according to the use as defined in claim 10.

12. Use according to claim 11, wherein the capture probe is directed against one or more epitopes located in amino acid positions 45-92 of Pre-pro-ADM.

## Patentansprüche

1. Verfahren zum Vorhersagen des Risikos, einen ersten nachteiligen Vorfall bei einem gesunden Individuum zu erleiden, oder zum Identifizieren eines gesunden Individuums mit einem erhöhten Risiko, einen ersten nachteiligen Vorfall zu erleiden, das Folgendes umfasst:
• Bestimmen des Spiegels an Pro-Adrenomedullin mit der Aminosäuresequenz SEQ ID NO:2 oder einem Fragment davon, das aus der Gruppe ausgewählt ist, bestehend aus PAMP, das SEQ ID NO:3 ist, MR-proADM, das SEQ ID NO:4 ist, und ADM, das SEQ ID NO:5 ist, in einer Probe, die von diesem Individuum erhalten wurde; und
• Verwenden des Spiegels an Pro-Adrenomedullin oder des Fragments davon für das Vorhersagen des ersten nachteiligen Vorfalls oder Ableiten eines Risikos davon, den ersten nachteiligen Vorfall zu erleiden,
wobei die Verwendung des Spiegels an Pro-Adrenomedullin oder dem Fragment davon den Vergleich des Spiegels an Pro-Adrenomedullin oder des Fragments davon mit einem vorbestimmten Schwellenwert umfasst, der das 75., 90., 95. oder 99. Perzentil einer normalen Population ist, wobei, wenn der Spiegel an Pro-Adrenomedullin oder dem Fragment davon den bestimmten Schwellenwert übersteigt, der erste nachteilige Vorfall bei dem gesunden Individuum vorhergesagt wird oder das gesunde Individuum mit einem erhöhten Risiko, den ersten nachteiligen Vorfall zu erleiden, identifiziert wird, und
wobei der erste nachteilige Vorfall ein koronarer oder kardiovaskulärer Vorfall ist, wobei der koronare Vorfall als tödliche oder nicht tödliche akute Koronarsyndrome definiert ist, einschließlich eines Myokardinfarkts oder des Todes aufgrund einer ischämischen Herzerkrankung, wobei der kardiovaskuläre Vorfall als tödliche oder nicht tödliche akute Koronarsyndrome definiert ist, einschließlich Myokardinfarkt, tödlicher oder nicht tödlicher Schlaganfall oder Tod aufgrund einer kardiovaskulären Erkrankung, und wobei das gesunde Individuum ein Individuum ist, das vorher keinen kardiovaskulären oder keinen koronaren Vorfall aufgewiesen hat und das keine akute Infektionskrankheit hat und das nicht an Diabetes und/oder Bluthochdruck leidet, und
wobei die Probe aus der Gruppe ausgewählt ist, bestehend aus einer Blutprobe, einer Serumprobe und einer Plasmaprobe.

2. Verfahren nach Anspruch 1, wobei der Spiegel an Pro-Adrenomedullin oder einem Fragment davon, das aus der Gruppe ausgewählt ist, bestehend aus PAMP, das SEQ ID NO: 3ist, MR-proADM, das SEQ ID NO:4 ist, und ADM, das SEQ ID NO:5 ist, bestimmt und als ein einzelner Marker verwendet wird.

3. Verfahren nach Anspruch 1, wobei das Vorhersagen des ersten nachteiligen Vorfalls bei dem gesunden Individuum oder das Identifizieren des gesunden Individuums mit einem erhöhten Risiko, den ersten nachteiligen Vorfall zu erleiden, verbessert wird, indem zusätzlich der Spiegel mindestens eines weiteren Markers bestimmt und verwendet wird, der aus der Gruppe ausgewählt ist, bestehend aus: CRP, LpLA2, Cystatin C, ANP, proANP, NT-proANP, MR-proANP, BNP, proBNP, NT-proBNP Triglyceride, HDL-Cholesterin oder Subfraktionen davon, LDL-Cholesterin oder Subfraktionen davon, GDF15, ST2, Procalcitonin, Pro-Vasopressin einschließlich Copeptin, Vasopressin und Neurophysin, Pro-Endothelin-1, CT-ProET-1, NT-ProET-1, Big-Endothelin-1 und Endothelin-1.

4. Verfahren nach Anspruch 3, wobei nur der Spiegel des folgenden Markers bestimmt und verwendet wird: proBNP, CRP oder LpLA2 in Kombination mit Pro-Adrenomedullin oder einem Fragment davon, das aus der Gruppe ausgewählt ist, bestehend aus PAMP, das SEQ ID NO:3 ist, MR-proADM, das SEQ ID NO:4 ist, und ADM, das SEQ ID NO:5 ist.

5. Verfahren nach Anspruch 4, wobei der erste kardiovaskuläre Vorfall bei dem gesunden Individuum vorhergesagt wird oder das gesunde Individuum mit erhöhtem Risiko, den ersten kardiovaskulären Vorfall zu erleiden, identifiziert wird.

6. Verfahren nach Anspruch 3, wobei nur der Spiegel des folgenden Markers bestimmt und verwendet wird: proBNP in Kombination mit Pro-Adrenomedullin oder einem Fragment davon, das aus der Gruppe ausgewählt ist, bestehend aus PAMP, das SEQ ID NO:3 ist, MR-proADM, das SEQ ID NO:4 ist, und ADM, das SEQ ID NO:5 ist.

7. Verfahren nach Anspruch 6, wobei der erste koronare Vorfall bei dem gesunden Individuum vorhergesagt wird oder das gesunde Individuum mit einem erhöhten Risiko, den ersten koronaren Vorfall zu erleiden, identifiziert wird.

8. Verfahren nach den Ansprüchen 1 bis 7, wobei zusätzlich mindestens ein klinischer Parameter bestimmt wird, der aus der Gruppe ausgewählt wird, die Folgendes umfasst: Alter, Geschlecht, antihypertensive Behandlung, Body-Mass-Index und aktuelles Rauchen.

9. Verfahren nach einem der vorangehenden Ansprüche, wobei der Spiegel an MR-proADM unter Verwendung eines diagnostischen Assays gemessen wird, der eine oder mehrere Fängersonde(n) (capture probe) umfasst, die gegen ein oder mehrere Epitop(e) gerichtet ist/sind, das/die sich in den Aminosäurepositionen 45-92 von Pre-pro-ADM befindet/befinden.

10. Verwendung eines MR-proADM-Assays in den Verfahren nach einem der Ansprüche 1 bis 9.

11. Verwendung einer Fängersonde, die gegen Pro-Adrenomedullin oder ein Fragment davon gerichtet ist, das aus der Gruppe ausgewählt ist, bestehend aus PAMP, das SEQ ID NO:3 ist, MR-proADM, das SEQ ID NO:4 ist, und ADM, das SEQ ID NO:5 ist, zum Vorhersagen des ersten nachteiligen Vorfalls bei dem gesunden Individuum oder zum Identifizieren des gesunden Individuums mit einem erhöhten Risiko, den ersten nachteiligen Vorfall zu erleiden, nach den Verfahren nach einem der Ansprüche 1 bis 9 oder nach der Verwendung wie in Anspruch 10 definiert.

12. Verwendung nach Anspruch 11, wobei die Fängersonde gegen ein oder mehrere Epitop(e) gerichtet ist/sind, das/die sich in den Aminosäurepositionen 45-92 von Pre-pro-ADM befindet/befinden.

## Revendications

1. Méthode pour prédire le risque de voir apparaître un premier événement indésirable chez un sujet sain ou identifier un sujet sain présentant un risque accru de voir apparaître un premier événement indésirable comprenant:
• la détermination du niveau de proadrénomédulline présentant la séquence d'acides aminés SEQ ID NO: 2 ou d'un fragment de celle-ci choisi dans le groupe consistant en PAMP qui est SEQ ID NO: 3, MR-proADM qui est SEQ ID NO: 4 et ADM qui est SEQ ID NO: 5 dans un échantillon prélevé sur ledit sujet; et
• l'utilisation dudit niveau de proadrénomédulline ou du fragment de celle-ci pour la prédiction du premier événement indésirable ou la déduction à partir de celui-ci d'un risque de voir apparaître le premier événement indésirable,
l'utilisation dudit niveau de proadrénomédulline ou du fragment de celle-ci comprenant la comparaison dudit niveau de proadrénomédulline ou du fragment de celle-ci à un niveau de seuil prédéfini étant le 75^{e}, 90^{e}, 95^{e} ou 99^{e} centile d'une population normale, au moyen duquel, lorsque ledit niveau de proadrénomédulline ou du fragment de celle-ci dépasse ledit niveau de seuil prédéfini, le premier événement indésirable est prédit chez le sujet sain ou le sujet sain présentant un risque accru de voir apparaître le premier événement indésirable est identifié et
ledit premier événement indésirable étant un événement coronaire ou cardiovasculaire, l'événement coronaire étant défini comme des syndromes coronariens aigus fatals ou non fatals incluant l'infarctus du myocarde, ou la mort due à une cardiopathie ischémique, un événement cardiovasculaire étant défini comme des syndromes coronariens aigus fatals ou non fatals incluant l'infarctus du myocarde, l'accident vasculaire cérébral fatal ou non fatal, ou la mort due à une maladie cardiovasculaire, et, le sujet sain étant un sujet qui n'a pas présenté préalablement d'événement cardiovasculaire ou coronarien et qui ne présente pas de maladie infectieuse aiguë et qui ne souffre pas de diabète et/ou d'hypertension et
ledit échantillon étant choisi dans le groupe consistant en un échantillon de sang, un échantillon de sérum et un échantillon de plasma.

2. Méthode selon la revendication 1, dans laquelle le niveau de proadrénomédulline ou d'un fragment de celle-ci choisi dans le groupe consistant en PAMP qui est SEQ ID NO: 3, MR-proADM qui est SEQ ID NO: 4 et ADM qui est SEQ ID NO: 5 est déterminé et utilisé comme un marqueur unique.

3. Méthode selon la revendication 1, dans laquelle la prédiction du premier événement indésirable chez le sujet sain ou l'identification du sujet sain présentant un risque accru de voir apparaître le premier événement indésirable est améliorée en déterminant et en utilisant en outre le niveau d'au moins un autre marqueur choisi dans le groupe consistant en: CRP, LpLA2, pystatine C, ANP, proANP, NT-proANP, MR-proANP, BNP, proBNP, NT-proBNP, des triglycérides, du cholestérol HDL ou des sous-fractions de celui-ci, du cholestérol LDL ou des sous-fractions de celui-ci, GDF15, ST2, procalcitonine, provasopressine incluant copeptine, vasopressine et neurophysine, proendothéline-1, CT-proET-1, NT-proET-1, big-endothéline-1 et endothéline-1.

4. Méthode selon la revendication 3, dans laquelle seul le niveau du marqueur suivant est déterminé et utilisé: proBNP, CRP ou LpLA2 en combinaison avec la proadrénomédulline ou un fragment de celle-ci choisi dans le groupe consistant en PAMP qui est SEQ ID NO: 3, MR-proADM qui est SEQ ID NO: 4 et ADM qui est SEQ ID NO: 5.

5. Méthode selon la revendication 4, dans laquelle le premier événement cardiovasculaire est prédit chez le sujet sain ou le sujet sain est identifié comme présentant un risque accru de voir apparaître le premier événement cardiovasculaire.

6. Méthode selon la revendication 3, dans laquelle seul le niveau du marqueur suivant est déterminé et utilisé: proBNP en combinaison avec la proadrénomédulline ou un fragment de celle-ci choisi dans le groupe consistant en PAMP qui est SEQ ID NO: 3, MR-proADM qui est SEQ ID NO: 4 et ADM qui est SEQ ID NO: 5.

7. Méthode selon la revendication 6, dans laquelle le premier événement coronaire est prédit chez le sujet sain ou le sujet sain est identifié comme présentant un risque accru de voir apparaître le premier événement coronaire.

8. Méthode selon les revendications 1 à 7, dans laquelle en outre au moins un paramètre clinique est déterminé choisi dans le groupe comprenant: âge, sexe, traitement antihypertenseur, indice de masse corporelle, et tabagisme actuel.

9. Méthode selon l'une des revendications précédentes, dans laquelle le niveau de MR-proADM est mesuré à l'aide d'un test de diagnostic comprenant une ou plusieurs sondes de capture dirigées contre un ou plusieurs épitopes situés dans les positions d'acides aminés 45 à 92 du préproADM.

10. Utilisation d'un essai de MR-proADM dans les méthodes de l'une des revendications 1 à 9.

11. Utilisation d'une sonde de capture dirigée contre la proadrénomédulline ou un fragment de celle-ci choisi dans le groupe consistant en PAMP qui est SEQ ID NO: 3, MR-proADM qui est SEQ ID NO: 4 et ADM qui est SEQ ID NO: 5 pour prédire le premier événement indésirable chez le sujet sain ou identifier le sujet sain présentant un risque accru de voir apparaître le premier événement indésirable selon les méthodes de l'une des revendications 1 à 9, ou selon l'utilisation telle que définie dans la revendication 10.

12. Utilisation selon la revendication 11, dans laquelle la sonde de capture est dirigée contre un ou plusieurs épitopes situés dans les positions d'acides aminés 45 à 92 du préproADM.
